# EUROPÄISCHE PATENTSCHRIFT

(11) **EP 2 269 695 B1**
(45) Veröffentlichungstag und Bekanntmachung des Hinweises auf die Patenterteilung: **21.12.2016**
(21) Anmeldenummer: 10006045.8
(22) Anmeldetag: 12.04.2006
(51) Int. Cl.: A61Q 5/06, A61K 8/81

(54) **Kosmetische Zubereitungen enthaltend eine Kombination aus einem Copolymer und mindestens einem amphoteren Polymer.**
Cosmetic compositions comprising a combination of a copolymer and at least one amphoteric polymer.
Compositions cosmétiques comprenant une combinaison d'un copolymère et d'au moins un polymère amphotérique

(30) Priorität: 14.04.2005 DE 102005017463
(43) Veröffentlichungstag der Anmeldung: 05.01.2011
(62) Teilanmeldung aus: 06112533.2
(73) Patentinhaber: Beiersdorf AG, 20253 Hamburg (DE)
(72) Erfinder: Argembeaux, Horst, 21465 Wentorf (DE); Detert, Marion, 22455 Hamburg (DE); Saß, Viola, 25488 Holm (DE); Franck, Kerstin, 25451 Quickborn (DE)

(56) Entgegenhaltungen:
- WO-A1-2005/082322
- WO-A1-2005/123014
- ANONYMOUS: "Wässrige Zubereitungen enthaltend wenigstens ein wasserlösliches oder wasserdispergierbares Copolymer mit kationogenen Gruppen" INTERNET ARTICLE, [Online] 7. März 2005 (2005-03-07), XP002389967 Gefunden im Internet: URL:http://pddocserv/specdocs/data/defensi ve_pubs/ip_com/2005/IPCOM000097478D/1_prim ary%20(2).pdf> [gefunden am 2010]
- Cognis Corporation: "Nutrilan I Powder" Technical Data Sheet 2000, XP002598412 Gefunden im Internet: URL:http://www.gmzinc.com/uploads/docs/TD_ NUTRILAN_%20I%20POWDER.pdf [gefunden am 2010-08-27]
- SORANA D. BOLBOACA, JÄNTSCHI L: "Amino acid Sequence Analysis on Collagen" BULLETIN OF UNIVERSITY OF AGRICULTURAL SCIENCES AND VETERINARY MEDICINE-CN, Bd. 63-64, 2007, Seiten 311-316, XP002598413 Romania ISSN: 1843-5262

## Beschreibung

Die vorliegende Erfindung betrifft eine kosmetische Zubereitung enthaltend eine Kombination aus einem Copolymer gebildet aus den Monomeren N-Vinylpyrrolidon (VP), N-Vinylimidazol (VI), Methacrylamid (MAM) und quaternisiertem N-Vinylimidazol (QVI) sowie einem Octylacrylamide/Acrylate/Butylaminoethyl Methacrylate Copolymer.

Der ganze menschliche Körper mit Ausnahme der Lippen, der Handinnenflächen und der Fußsohlen ist behaart, zum Großteil allerdings mit kaum sichtbaren Wollhärchen. Wegen der vielen Nervenden an der Haarwurzel reagieren Haare empfindlich auf äußere Einflüsse wie Wind oder Berührung und sind daher ein nicht zu unterschätzender Bestandteil des Tastsinns. Die wichtigste Funktion des menschlichen Kopfhaares dürfte allerdings heute darin bestehen, das Aussehen des Menschen in charakteristischer Weise mitzugestalten. Ähnlich wie die Haut erfüllt es eine soziale Funktion, da es über sein Erscheinungsbild erheblich zum Selbstwertgefühl des Individuums beiträgt.

Das Haar besteht aus dem frei aus der Haut herausragenden Haarschaft - dem keratinisierten (toten) Teil, der das eigentlich sichtbare Haar darstellt - und der in der Haut steckenden Haarwurzel - dem lebenden Teil, in dem das sichtbare Haar ständig neu gebildet wird. Der Haarschaft seinerseits ist aus drei Schichten aufgebaut: einem zentralen Teil - dem so genannten Haarmark (Medulla), welches allerdings beim Menschen zurückgebildet ist und oft gänzlich fehlt - ferner dem Mark (Cortex) und der äußeren, bis zu zehn Lagen starken Schuppenschicht (Cuticula), die das ganze Haar umhüllt.

Natürliches Haar hängt meist schlaff und ohne Volumen vom Kopf herab. Ein Ziel der Haarpflege ist es daher, dem Haar Fülle und Frisur zu geben. Zur temporären Verformung des Haares und Gestaltung (engl. Styling) vielseitiger Frisuren verwendet man Haarfestiger, meist in Form von Schaumfestigern oder Haarsprays. Beide haarkosmetischen Mittel sind in Ihrer Zusammensetzung ähnlich unterscheiden sich aber in ihrer Aufgabe und Anwendung. Schaumfestiger (auch Schaumhaarfestiger genannt) werden in der Regel zur Frisurgestaltung im feuchten Haar verteilt, Haarsprays zur Fixierung auf die (trockene) fertig gestylte Frisur gesprüht. Neben den wegen ihrer leichten und angenehmen Anwendbarkeit zunehmend beliebten Schaumhaarfestigern und flüssigen Haarfestigern werden auch Haarfestigergele angeboten.

Die Rezepturen für derartige Produkte sind vielfältig. Die Bestandteile müssen jedoch in ethanolischem, wässrig-ethanolischem oder wässrigem Medium verträglich sein.

Üblicherweise bestehen diese Mittel zur Festigung der Haare (Haarfestiger) aus Lösungen von filmbildenden natürlichen oder synthetischen Polymeren oder Polymerkombinationen. Es handelt sich dabei um nichtionische, anionische, kationische oder amphotere Polymere. Diese hinterlassen auf dem Haar nach der Applikation einen transparenten farblosen Film. Es werden eine Reihe von Anforderungen an diese Filme gestellt: sie sollen klar, glänzend, feuchtigkeitsunempfindlich, festigend, lang anhaltend, flexibel, nicht klebend sein, den natürlichen Griff des Haares nicht beeinträchtigen und sich auch wieder leicht durch handelsübliche Haarwaschmittel auswaschen lassen. Schaumfestiger besitzen in der Gruppe der Stylingprodukte eine Sonderstellung. Nicht nur die Filmeigenschaften (Halt, Flexibilität...) stehen im Vordergrund, sondern auch die Pflegeleistung am Haar (Kämmbarkeit, gepflegter Griff) und die Schaumcharakteristik (feinblasig, cremig, stabil) haben einen beträchtlichen Einfluss auf die Produktperformance.

Kationische Filmbildner weisen vor allem pflegende Eigenschaften auf: das Haar lässt sich gut kämmen und fühlt sich geschmeidig und gepflegt an. Insbesondere für Schaumfestiger hat die Kämmbarkeit und der Griff der nassen und trockenen Haare eine entscheidende Bedeutung. Als entscheidender Nachteil dieser Rohstoffe ist ihre geringe Festigungsleistung zu nennen.

Die Verwendung von Mischungen kationischer Filmbildner zur Produktoptimierung von haarkosmetischen Zubereitungen ist dem Fachmann an sich bekannt.

Anionische oder amphotere Filmbildner werden in Haarfestigern eingesetzt, um eine gute und lang anhaltende Festigung der Frisur zu erreichen. Ein entscheidender Nachteil dieser Rohstoffe sind jedoch ihre mangelhaften pflegenden Eigenschaften: das Haar lässt sich schlecht kämmen und fühlt sich stumpf, rau und wenig gepflegt an. Nach dem Trocknen sind die Filme dann relativ hart, spröde und unflexibel. Nachteilig für den Verbraucher sind die taktilen Eigenschaften des Filmes sowie die Bildung von Rückständen beim Kämmen (Filmplaken). Diese rieseln auf Schultern oder Kleidung und lassen den Anwender ungepflegt erscheinen. Bei Schaumfestigern kommt darüber hinaus ein weiterer Nachteil vieler anionischer und amphoterer Polymere zum Tragen: sie verleihen dem Schaum eine unzureichende, unkosmetische Schaumcharakteristik. Der Schaum ist grobblasig, weich, instabil und wenig cremig. Zur Verbesserung der Schaumeigenschaften ist der Zusatz von Schaum stabilsierenden Additiven, wie x.B. Emulgataren üblich. Besonders bevorzugt ist der Einsatz von Laureth-3.

Eine Verbesserung in Bezug auf Griff und Kämmbarkeit wird bei Styling-Formulierungen auf Basis anionischer und amphotere Polymere typischerweise durch die Kombination mit kationischen Polymeren, kationischen Tensiden oder Silikonen (beispielsweise acyclische Polydimethylsiloxane (Cyclomethicone), Silikontenside (Polyethermodifizierte Siloxane) vom Typ Dimethicone) erreicht. Bei derartigen Zubereitungen besteht jedoch immer die Gefahr einer Ausfällung der Polymeren durch die Bildung von Polymersalzen aus anionischen und kationischen Polymeren. Außerdem führt der Einsatz dieser kationischen Polymere oder kationischen Tenside meistens zu einer deutliche Abnahme der Fertigungsleistung und des Langzeithaltes der Frisur,

Es war daher die Aufgabe der vorliegenden Erfindung, die Mängel des Standes der Technik zu beseitigen und Zubereitungen (insbesondere Mittel zur Frisurgestaltung) zu entwickeln, die zu einer guten Kämmbarkeit des Haares führen. Das Haar sollte sich nach der Anwendung nicht mehr stumpf anfühlen, sondern einen glatten, gut gepflegten Eindruck hinterlassen. Die Bildung von Filmplaken sollte unterdrückt sein. Festigungsleistung und Langzeithalt sollten gegenüber dem Stand der Technik erhöht sein. Darüber hinaus sollte sich das Produkt zu einem feinblasigen, cremigen, stabilen Schaum aufschäumen lassen.

Überraschend gelöst werden die Aufgaben durch einen Haarfestiger enthaltend eine Kombination aus gemäß Anspruch 1.

Zwar kennt der Fachmann die Veröffentlichung "wässrige Zubereitungen enthaltend wenigstens ein wasserlösliches oder wasserdispergierbares Copolymer mit kationogenen Gruppen", vom 7.März 2005, http://pddocserv/specdocs/data/defensive pubs/ip com/2005/IPCOM000097478/D1 primary %20(2).pdf, WO 2005/123014 und WO 2005/082322, doch konnten diese Schriften nicht den Weg zur vorliegenden Erfindung weisen.

Die erfindungsgemäßen Zubereitungen führen zu einer deutlichen Verbesserung der Pflegeleistung unter gleichzeitiger Erhöhung der Festigungsleistung bei ihrer Anwendung als Haarfestiger.

Der Langzeithalt der Frisur gemessen im "Curl-Retention-Test" bei 90 % r.F. ist deutlich erhöht (siehe Vergleichsversuch).

Liegen die erfindungsgemäßen Zubereitungen in Form eines Schaumfestigers vor, so weist die Zubereitung einen besonders cremigen, feinblasigen Schaum auf der sich überraschend leicht und gleichmäßig im Haar verteilen lässt.

Das Haar lässt sich nach der Anwendung besonders leicht kämmen und weist einen gesunden, seidigen Glanz auf. Die Bildung von aus dem Haar herab rieselnden Filmplaken ist deutlich reduziert.

Zwar ist dem Fachmann der Einsatz von einem Copolymer gebildet aus den Monomeren N-Vinylpyrrolidon (VP), N-Vinylimidazol (VI), Methacrylamid (MAM) und quaternisiertem N-Vinylimidazol (QVI) in kosmetischen Zubereitungen an sich bekannt. So offenbart der "Technical Disclosure" "Wässrige Zubereitungen enthaltend wenigstens ein wasserlösliches oder wasserdispergierbares Copolymer mit kationogenen Gruppen", veröffentlicht in "IP.com" Prior Art Database vom 16.3.2005 (link: http://ip.com/pubView/IPCOM000097478D) kosmetische Zubereitungen mit derartigen Polymeren. Doch konnte diese Veröffentlichung nicht den Weg zur vorliegenden Erfindung weisen, da keine Möglichkeiten zur Verbesserung der Halte- oder Pflegeleistung von haarkosmetischen Zubereitungen beschrieben wurden.

Erfindungsgemäß vorteilhafte Ausführungsformen der Erfindung sind dadurch gekennzeichnet, dass die Zubereitung das Copolymer aus den Monomeren N-Vinylpyrrolidon (VP), N-Vinylimidazol (VI), Methacrylamid (MAM) und quaternisiertem N-Vinylimidazol (QVI) in einer Konzentration von 0,01 bis 10 Gewichts-% und bevorzugt in einer Konzentration von 2,5 bis 7,5 Gewichts-% (tel quel), jeweils bezogen auf das Gesamtgewicht der Zubereitung, enthält.

Es ist erfindungsgemäß vorteilhaft, wenn die Zubereitung anionische und amphotere Polymere in einer Gesamtkonzentration von 0,01 bis 20 Gewichts-% und bevorzugt in einer Gesamtkonzentration von 2 bis 10 Gewichts-% (tel quel), jeweils bezogen auf das Gesamtgewicht der Zubereitung, enthält.

Es ist erfindungsgemäß vorteilhaft, wenn das Gewichtsverhältnis von Copolymer aus den Monomeren N-Vinylpyrrolidon (VP), N-Vinylimidazol (VI), Methacrylamid (MAM) und quaternisiertem N-Vinylimidazol (QVI) zur Gesamtmenge aus anionischen und amphoteren Polymeren 5:1 bis 1:5 beträgt (bevorzugt: 1:1 bis 1:4).

Eine erfindungsgemäß besonders bevorzugte Ausführungsform der vorliegenden Erfindung ist dadurch gekennzeichnet, dass das Copolymer aus N-Vinylpyrrolidon (VP), N-Vinylimidazol (VI), Methacrylamid (MAM) und quaternisiertem N-Vinylimidazol (QVI) die Monomere N-Vinylpyrrolidon (VP), N-Vinylimidazol (VI), Methacrylamid (MAM) und quaternisiertem N-Vinylimidazol (QVI) in einem Gewichtsverhältnis von 55:10:29:6 enthält. Dabei können die einzelnen Mengenbestandteile herstellungsbedingt schwanken. Abweichungen von 10 % sind als erfindungsgemäß anzusehen.

Vorteilhaft im Sinne der vorliegenden Erfindung ist es, wenn ein oder mehrere anionische Polymere gewählt werden aus der Gruppe der Verbindungen Acrylat / C1-2 Succinat / Hydroxyacrylat-Copolymere; Vinylacetat/Crotonsäure-, Vinylacetat/Acrylat- und/oder Vinylacetat/Vinylneodecanoat/Crotonsäure-Copolymere, Natriumacrylat/Vinylalkohol-Copolymere, Natriumpolystyrolsulfonat, Ethylacrylat/N-tert.-Butylacrylamid/Acrylsäure-Copolymere, Vinylpyrrolidon/Vinylacetat/Itaconsäure-Copolymere, Acrylsäure/Acrylamid-Copolymere und/oder deren Natriumsalze, Homo- und/oder Copolymere von Acrylsäure und/oder Methacrylsäure und/oder deren Salze sowie Acrylat/Hydroxyacrylat-, Octylacrylamid/Acrylat- bzw. Methacrylsäurester und/oder Butylacrylat/N-Vinylpyrrolidon-Copolymere, Methylvinylether/Maleinsäure-Copolymere, die durch Hydrolyse von Vinylether/Maleinsäureanhydrid-Copolymeren entstehen sowie deren Ethyl, Isopropyl- bzw. Butylester.

Dabei ist es erfindungsgemäß besonders bevorzugt, wenn als anionisches Polymer Acrylat / C1-2 Succinat/Hydroxyacrylat-Copolymer (INCI: Acrylates/C1-2 Succinates/Hydroxyacrylates Copolymer) eingesetzt wird, welches z.B. bei der Firma ISP unter dem Handelsnamen Allianz LT-120 erhältlich ist.

Erfindungsgemäße Ausführungsformen stellen Zubereitungen dar, die dadurch gekennzeichnet sind, dass ein oder mehrere amphoteren Polymere gewählt werden aus der Gruppe der Verbindungen Polyimide-1 (Isobuthylene/dimethylaminopropyl-maleimide/ethoxylated maleimide/maleate ester copolymer), Octylacrylamide/Acrylate/Butylaminoethyl Methacrylate Copolymer, Acrylates/Lauryl Acrylate/Stearyl Acrylate/Ethylamine Oxide Methacrylate Copolymer, Methacryloyl Ethylbetaine/Methacrylates Copolymer, Copolymerisate aus Carboxylgruppen oder Sulfongruppen enthaltenden Monomeren, z.B. (Meth)Acrylsäure und Itaconsäure mit basischen insbesondere Aminogruppen enthaltenden Monomeren wie z.B. Mono- bzw. Dialkylamino-alkyl(meth)acrylaten und/oder Mono- bzw. Dialkylaminoalkyl(meth)acrylamiden.

Dabei ist es errindungsgemäß besonders bevorzugt, wenn als amphoteres Polymer Isobuthylene / dimethylaminopropylmaleimide / ethoxylated maleimide / maleate ester copolymer (INCI: Polyimide-1) eingesetzt wird, welches z.B. bei der Firma ISP unter dem Handelsnamen Aquaflex XL-30 erhältlich ist.

Die erfindungsgemäßen Zubereitungen können erfindungsgemäß vorteilhaft weitere kationische Polymere und/oder kationische Tenside in einer Gesamtkonzentration von 0,1 bis 5 Gewichts-%, bezogen auf das Gesamtgewicht der Zubereitung, enthalten.

Als erfindungsgemäß vorteilhafte kationische Polymere können Polyquaternium-4, Polyquaternium-6, Polyquaternium-7, Polyquaternium-10, Polyquaternium-11, Polyquaternium-16, Polyquaternium-22, Polyquaternium-44, Polyquaternium-46, Polyquaternium-55 und/oder quaternisierte Guarderivate eingesetzt werden.

Als erfindungsgemäß vorteilhafte kationische Tenside können Verbindungen aus der Gruppe der quaternären Ammoniumverbindungen, insbesondere Benzyltrialkylammoniumsalze oder Alkyltrimethylammoniumsalze, beispielsweise Cetyltrimethylammoniumchlorid oder -bromid oder -methosulfat, Alkyldimethylhydroxyethylammoniumchloride oder -bromide oder - methosulfat, Dialkyldimethylammonium-chloride oder -bromide oder -methosulfat, Esterquats wie z.B. Diacylethyl Hydroxyethylmethylammoniumchlorid oder -bromid oder - methosulfat, Alkylamidethyltrimethylammoniumethersulfate, Hydroxyethyl Cetyldimonium Phosphate, Alkylpyridiniumsalze, beispielsweise Lauryl- oder Cetylpyrimidiniumchlorid, Imidazolinderivate und Verbindungen mit kationischem Charakter wie Aminoxide.
beispielsweise Alkyldimethylaminoxide oder Alkylaminoethyl-dimethylaminoxide eingesetzt werden.

Es ist erfindungsgemäß vorteilhaft, wenn die erfindungsgemäße Zubereitung mit einem Treibgas aufgeschäumt ist. Das Treibgas wird dabei erfindungsgemäß vorteilhaft in einer Menge von 0,5 bis 20 Gewichts-%, besonders vorteilhaft in einer Menge von 5 bis 15 und ganz besonders vorteilhaft in einer Menge von 8 bis 11 Gewichts-%, jeweils bezogen auf das Gesamtgewicht der Formulierung eingesetzt. Die Verwendung einer Pumpschäumvorrichtung für treibmittelfreie Schaumfestiger ist ebenfalls möglich.

Zum Aufschäumen der Zubereitung werden dabei erfindungsgemäß bevorzugt Propan/Butan-Gemische eingesetzt.

Natürlich weiß der Fachmann, dass es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Eine besonders bevorzugte Ausführungsform der erfindungsgemäßen Zubereitung ist ihr Vorliegen in Form einer wässrigen oder wässrig-alkoholischen Lösung.

Erfindungsgemäß vorteilhaft enthält die erfindungsgemäße Zubereitung weitere kosmetische Hilfsstoffe z.B. Pflegestoffe, Konservierungsmittel, Bakterizide, Parfüme, Emulgatoren, weichmachende, anfeuchtende und/oder feuchthaltende Substanzen, Fette, Öle, Wachse, Fettalkohole, Fettsäureester oder andere übliche Bestandteile einer kosmetischen oder dermatologischen Formulierung wie Alkohole, Polyole, Polymere, Schaumstabilisatoren, Elektrolyte, organische Lösungsmittel oder Siliconderivate. Auch die Einarbeitung von UV-Lichtschutzfiltern ist erfindungsgemäß vorteilhaft. Die aufgeführte Liste an Zusatzstoffen soll selbstverständlich nicht limitierend sein.

Erfindungsgemäß vorteilhaft enthält die erfindungsgemäße Zubereitung 0,1 bis 0,4 Gewichts-%, bezogen auf das Gesamtgewicht der Zubereitung, Zitronensäure und/oder deren Salze.

Vorteilhaft im Sinne der vorliegenden Erfindung ist es , der erfindungsgemäßen Zubereitung Panthenol zuzusetzen. Der Gehalt an Panthenol beträgt dabei erfindungsgemäß vorteilhaft 0,01 bis 0,3 Gewichts-%, bezogen auf das Gesamtgewicht der Zubereitung.

Erfindungsgemäß vorteilhaft enthält die erfindungsgemäße Zubereitung einen oder mehrere O/W-Emulgatoren. Dabei ist insbesondere der Einsatz von Polyethylene Glycol 2000 Hydrogenated Castor Oil (INCI: PEG-40 Hydrogenated Castor Oil) erfindungsgemäß besonders bevorzugt. Erfindungsgemäß vorteilhaft hat sich dabei eine Einsatzkünzentration von 0,05 bis 0,5 Gewichts-%, bezogen auf das Gesamtgewicht der Zubereitung, erwiesen.

Erfindungsgemäß vorteilhaft enthält die erfindungsgemäße Zubereitung Cetyltrimethylammoniumchlorid (Cetrimonium chloride, CAS 112-02-7), welches bevorzugt in einer Konzentration von 0,1 bis 2 Gewichts-%, bezogen auf das Gesamtgewicht der Zubereitung in dieser enthalten ist.

Erfingdungsgemäß vorteilhaft einhält die erfindungsgemäße Zubereitung Laureth-3. Dieses wird erfindungsgemäß bevorzugt in einer Konzentration von 0,5 bis 3 Gewichts-%, bezogen auf das Gesamtgewicht der Zubereitung, eingesetzt. Laureth-3 kann unter den folgenden Handelsnamen bei den genannten Rohstoffherstellern bezogen werden; Dehydol LS 3 Deo N (Cognis), AEC Laureth-3 (A & E Connock), Empilan KB 3 (Albright & Wilson), Genapol L-3 (Clariant), Emalex 703 (Nihon Emulsion), Glycolene (Vevy), Hetoxol L-3N (Heterene), Jeecol LA-3 (Jeen), Marlipal 24/30 (Sasol). Sabowax LM 3 (Sabo), Sympathens-ALM/030 (Kolb), Unihydol LS-3 (Universal Preserv-A-Chem).

Erfindungsgemäß besonders bevorzugt ist die Verwendung der erfindungsgemäßen Zubereitung als Schaumfestiger für die Frisurgestaltung.

Das nachfolgende Beispiel soll die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

**Rezepturbeispiel:**

| | 1 |
|---|---|
| Copolymer aus N-Vinylpyrrolidon (VP), N-Vinylimidazol (VI), Methacrylamid (MAM) und quaternisiertem N-Vinylimidazol (QVI) | 7 |
| Octylacrylamide/ Acrylates/ Butylaminoethyl Methacrylate Copolymer | 3 |
| Aminomethylpropanol | 0,4 |
| Cetrimoniumchlorid | 1,0 |
| Hydroxyethyl Cetyldimonium Phosphate | 0,5 |
| Dimethicone Copolyol | 0,2 |
| Panthenol | 0,1 |
| Laureth-3 | 2,0 |
| PEG-40 Hydrogenated Castor Oil | 0,2 |
| Parfüm | q.s. |
| Propan/Butan | 10 |
| Ethanol | 15 |
| Wasser | ad 100 |

## Patentansprüche

1. Haarfestiger enthaltend eine Kombination aus
a) einem Copolymer gebildet aus den Monomeren N-Vinylpyrrolidon (VP), N-Vinylimidazol (VI), Methacrylamid (MAM) und quaternisiertern N-Vinylimidazol (QVI) sowie
b) Octylacrylamide/Acrylate/Butylaminoethyl Methacrylate Copolymer.

2. Haarfestiger nach Anspruch 1, **dadurch gekennzeichnet, dass** die Zubereitung das Copolymer a) in einer Konzentration von 0,01 bis 10 Gewichts-%, bezogen auf das Gesamtgewicht der Zubereitung, enthält.

3. Haarfestiger nach einem der Ansprüche 1 oder 2, **dadurch gekennzeichnet, dass** die Zubereitung anionische Polymere enthält.

4. Haarfestiger nach einem der vorhergehenden Ansprüche, **dadurch gekennzeichnet, dass** das Gewichtsverhältnis von Copolymer a) zur Gesamtmenge von anionischen und amphoteren Polymeren von 5:1 bis 1:5 beträgt.

5. Haarfestiger nach einem der vorhergehenden Ansprüche, **dadurch gekennzeichnet, dass** das Copolymer a) die Monomere N-Vinylpyrrolidon (VP), N-Vinylimidazol (VI), Methacrylamid (MAM) und quaternisiertem N-Vinylimidazol (QVI) in einem Gewichtsverhältnis von 55:10:29:6 enthält.

6. Haarfestiger nach Anspruch 3, **dadurch gekennzeichnet, dass** ein oder mehrere anionische Polymere gewählt werden aus der Gruppe der Verbindungen Acrylat / C1-2 Succinat / Hydroxyacrylat-Copolymer; Vinylacetat/Crotonsäure-, Vinylacetat/Acrylat- und/oder Vinylacetat/Vinylneodecanoat/Crotonsäure-Copolymere, Natriumacrylat/Vinylalkohal-Capolymere, Natriumpolystyrolsulfonat, Ethylacrylat/N-tert.-Butylacrylamid/Acrylsäure-Copalymere, Vinylpyrrolidon/Vinylacetat/Itaconsäure-Copolymere, Acrylsäure/Acrylamid-Copolymere und/oder deren Natriumsalze, Homo- und/oder Copolymere von Acrylsäure und/oder Methacrylsäure und/oder deren Salze sowie Acrylat/Hydroxyacrylat-, Octylacrylamid/Acrylat- bzw. Methacrylsäurester und/oder Butylacrylat/N-Vinylpyrrolidon-Copolymere, Methylvinylether/MaleinsäureCopolymere, die durch Hydrolyse von Vinylether/Maleinsäureanhydrid-Copolymeren entstehen sowie deren Ethyl, Isopropyl- bzw. Butylester.

7. Haarfestiger nach Anspruch 3-6, **dadurch gekennzeichnet, dass** als anionisches Polymer Acrylat / C1-2 Succinat / Hydroxyacrylat-Copolymer (INCI: Acrylates/C1-2 Succinates/Hydroxyacrylates Copolymer) eingesetzt wird.

8. Haarfestiger nach Anspruch 1-7, **dadurch gekennzeichnet, dass** als weiteres amphoteres Polymer Isobuthylene/dimethylaminopropyl-maleimide/ethoxylated maleimide/maleate ester copolymer (INCI: Polyimide-1) eingesetzt wird.

9. Haarfestiger nach einem der vorhergehenden Ansprüche, **dadurch gekennzeichnet, dass** die Zubereitung mit einem Treibgas aufgeschäumt ist.

## Claims

1. Hair-setting composition comprising a combination of
a) a copolymer formed from the monomers N-vinylpyrrolidone (VP), N-vinylimidazole (VI), methacrylamide (MAM) and quaternized N-vinylimidazole (QVI) and
b) octylacrylamide/acrylates/butylaminoethyl methacrylate copolymer.

2. Hair-setting composition according to Claim 1, **characterized in that** the preparation comprises the copolymer a) at a concentration of 0.01 to 10% by weight, based on the total weight of the preparation.

3. Hair-setting composition according to either of Claims 1 and 2, **characterized in that** the preparation comprises anionic polymers.

4. Hair-setting composition according to any of the preceding claims, **characterized in that** the ratio by weight of copolymer a) to the total amount of anionic and amphoteric polymers is from 5:1 to 1:5.

5. Hair-setting composition according to any of the preceding claims, **characterized in that** the copolymer a) comprises the monomers N-vinylpyrrolidone (VP), N-vinylimidazole (VI), methacrylamide (MAM) and quaternized N-vinylimidazole (QVI) in a ratio by weight of 55:10:29:6.

6. Hair-setting composition according to Claim 3, **characterized in that** one or more anionic polymers are selected from the group of compounds comprising acrylate / C1-2 succinate / hydroxyacrylate copolymer; vinyl acetate/crotonic acid, vinyl acetate/acrylate and/or vinyl acetate/vinyl neodecanoate/crotonic acid copolymers, sodium acrylate/vinyl alcohol copolymers, sodium polystyrene sulphonate, ethyl acrylate/N-tert-butylacrylamide/acrylic acid copolymers, vinylpyrrolidone/vinyl acetate/itaconic acid copolymers, acrylic acid/acrylamide copolymers and/or sodium salts thereof, homopolymers and/or copolymers of acrylic acid and/or methacrylic acid and/or salts thereof and also acrylate/hydroxyacrylate, octylacrylamide/acrylate or methacrylic ester and/or butyl acrylate/N-vinylpyrrolidone copolymers, methyl vinyl ether/maleic acid copolymers, which are produced by hydrolysis of vinyl ether/maleic anhydride copolymers and ethyl, isopropyl or butyl esters thereof.

7. Hair-setting composition according to Claims 3-6, **characterized in that** the anionic polymer used is acrylate / C1-2 succinate / hydroxyacrylate copolymer (INCI: Acrylates/C1-2 Succinates/Hydroxyacrylates copolymer).

8. Hair-setting composition according to Claims 1-7, **characterized in that** the further amphoteric polymer used is isobutylene/dimethylaminopropylmaleimide/ethoxylated maleimide/maleate ester copolymer (INCI: Polyimide-1).

9. Hair-setting composition according to any of the preceding claims, **characterized in that** the preparation is foamed using a propellant.

## Revendications

1. Fixateur pour cheveux, contenant une combinaison
a) d'un copolymère formé à partir des monomères N-vinylpyrrolidone (VP), N-vinylimidazole (VI), méthacrylamide (MAM) et N-vinylimidazole quaternisé (QVI) et
b) de copolymère d'octylacrylamide/acrylate/méthacrylate de butylaminoéthyle.

2. Fixateur pour cheveux selon la revendication 1, **caractérisé en ce que** la préparation contient le copolymère a) en une concentration de 0,01 à 10% en poids par rapport au poids total de la préparation.

3. Fixateur pour cheveux selon l'une quelconque des revendications 1 ou 2, **caractérisé en ce que** la préparation contient des polymères anioniques.

4. Fixateur pour cheveux selon l'une quelconque des revendications précédentes, **caractérisé en ce que** le rapport pondéral de copolymère a) à la quantité totale de polymères anioniques et amphotères est de 5:1 à 1:5.

5. Fixateur pour cheveux selon l'une quelconque des revendications précédentes, **caractérisé en ce que** le copolymère a) contient les monomères N-vinylpyrrolidone (VP), N-vinylimidazole (VI), méthacrylamide (MAM) et N-vinylimidazole quaternisé (QVI) en un rapport pondéral de 55:10:29:6.

6. Fixateur pour cheveux selon la revendication 3, **caractérisé en ce qu'**un ou plusieurs polymères anioniques sont choisis dans le groupe des composés copolymère d'acrylate/succinate de C1-2/hydroxyacrylate ; copolymères d'acétate de vinyle/acide crotonique, acétate de vinyle/acrylate et/ou acétate de vinyle/ néodécanoate de vinyle/acide crotonique, copolymères d'acrylate de sodium/alcool vinylique, polystyrènesulfonate de sodium, copolymères d'acrylate d'éthyle/N-tert-butylacrylamide/acide acrylique, copolymères de vinylpyrrolidone/acétate de vinyle/acide itaconique, copolymères d'acide acrylique/acrylamide et/ou leurs sels sodiques, homopolymères et/ou copolymères d'acide acrylique et/ou d'acide méthacrylique et/ou leurs sels ainsi que copolymères d'acrylate/hydroxyacrylate, copolymères. d'octylacrylamide/acrylate ou ester d'acide méthacrylique et/ou acrylate de butyle/N-vinylpyrrolidone, copolymères de méthylvinyléther/acide maléique, qui sont formés par hydrolyse de copolymères de vinyléther/anhydride de l'acide maléique ainsi que leurs esters d'éthyle, d'isopropyle ou de butyle.

7. Fixateur pour cheveux selon la revendication 3-6, **caractérisé en ce qu'**on utilise, comme polymère anionique, un copolymère d'acrylate/succinate de C1-2/hydroxyacrylate (INCI : Acrylates/C1-2 Succinates/Hydroxyacrylates Copolymer).

8. Fixateur pour cheveux selon la revendication 1-7, **caractérisé en ce qu'**on utilise, comme autre polymère amphotère, un copolymère d'isobutylène/diméthylaminopropylmaléimide/maléimide éthoxylé/ester de maléate (INCI : Polyimide-1).

9. Fixateur pour cheveux selon l'une quelconque des revendications précédentes, **caractérisé en ce que** la préparation est moussée avec un gaz propulseur.
